# EUROPÄISCHE PATENTANMELDUNG

(11) **EP 1 375 561 A1**
(43) Veröffentlichungstag der Anmeldung: **02.01.2004**
(21) Anmeldenummer: 03013601.4
(22) Anmeldetag: 16.06.2003
(51) Int. Cl.: C08G 73/02, C07C 209/78, C07C 263/10, C08G 18/32, C08G 18/76

(54) **Verfahren zur Herstellung von Polyisocyanaten der Diphenylmethanreihe mit vermindertem Farbwert**

(30) Priorität: 27.06.2002 DE 10228734
(71) Anmelder: Bayer Aktiengesellschaft, 51368 Leverkusen (DE)
(72) Erfinder: Hagen, Torsten, Dr., 45133 Essen (DE); Kämper, Friedhelm, Dr., 47807 Krefeld (DE); Koch, Daniel, Dr., 47137 Duisburg (DE); Müller, Heinz-Herbert, Dr., 47809 Krefeld (DE)

(57) **Zusammenfassung**

Die Erfindung betrifft ein Verfahren zur Herstellung von Polyisocyanaten der Diphenylmethanreihe, bei dem man
a) Anilin und Formaldehyd in Gegenwart eines sauren Katalysators zu Polyaminen umsetzt und danach
b) das Reaktionsgemisch in Gegenwart eines Alkohols neutralisiert und /oder das Reaktionsgemisch nach der Neutralisation mit einem Alkohol versetzt und /oder nach der Neutralisation die Phasen trennt und die organische Phase mit einer Base und einem Alkohol versetzt,
wobei das molare Verhältnis des Alkohols zum eingesetzten Formaldehyd mindestens 0,02 ist und danach
c) die Polyamine durch Phosgenierung zu den entsprechenden Polyisocyanaten umsetzt.

## Beschreibung

Die Erfindung betrifft ein Verfahren zur Herstellung von Polyaminen der Diphenylmethanreihe sowie ein Verfahren zur Herstellung von Polyisocyanaten der Diphenylmethanreihe mit verminderten Farbwerten, welche durch Umsetzung der entsprechenden Polyamine der Diphenylmethanreihe mit Phosgen gewonnen werden.

Unter Polyisocyanaten der Diphenylmethanreihe werden Isocyanate und Isocyanatgemische folgenden Typs verstanden:

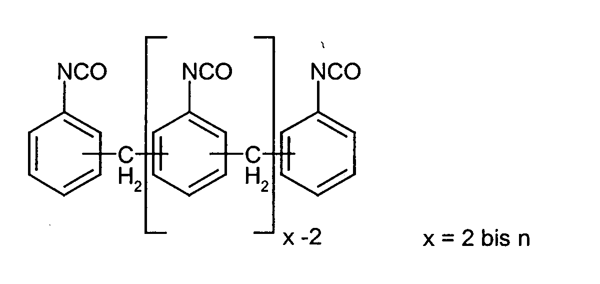

Analog werden unter Polyaminen der Diphenylmethanreihe Verbindungen und Verbindungsgemische folgenden Typs verstanden: 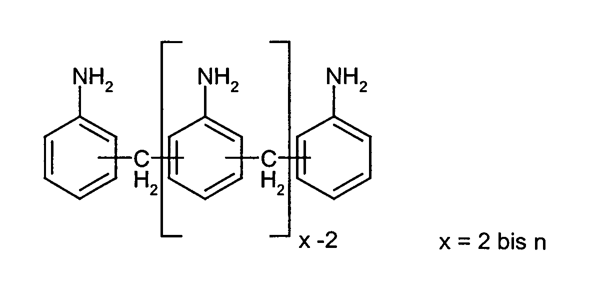 Die großtechnische Herstellung von Isocyanaten durch Umsetzung von Aminen mit Phosgen in Lösungsmitteln ist bekannt und in der Literatur ausführlich beschrieben (Ullmanns Enzyklopädie der technischen Chemie, 4. Auflage, Band 13, Seite 347-357, Verlag Chemie GmbH, Weinheim, 1977). Auf Basis dieses Verfahrens werden Polyisocyanatgemische hergestellt, die als Polyisocyanatkomponenten bei der Herstellung von Polyurethanschäumen und anderen nach dem Polyadditionsverfahren hergestellten Polyurethankunststoffen dienen.

Es ist allgemein bekannt, dass bei diesem Verfahren auch unerwünschte Farbstoffe oder farbgebende Komponenten gebildet werden, die auch bei der Weiterverarbeitung zu Polyurethanschäumen oder anderen Polyurethankunststoffen erhalten bleiben. Obwohl die Eigenfarbe der Polyisocyanat-Polyadditionsprodukte deren mechanische Eigenschaften nicht negativ beeinflusst, werden vom Verbraucher im wesentlichen farblose Produkte gewünscht. Als Maß für die Verfärbung des Polyisocyanates dient die Extinktion bei verschiedenen Wellenlängen.

Daher ist seit längerer Zeit die Reduzierung der Farbwerte von Polyisocyanaten der Diphenylmethanreihe das Ziel zahlreicher Versuche und Arbeiten, die in der Literatur beschrieben sind. So beschreibt beispielsweise DE-A1-4208359 die Behandlung von solchen Isocyanaten mit Wasserstoff in Gegenwart von Trägerkatalysatoren. DE-A1-4232769 beschreibt die Zugabe von Aminen, Harnstoffen und Antioxidantien zum Isocyanat. DE-A1-19815055 beschreibt die Farbverbesserung von Polyisocyanaten der Diphenylmethanreihe durch Bestrahlung mit Licht über einen längeren Zeitraum. DE-A1-19804915 beschreibt die Aufhellung von Polyisocyanaten der Diphenylmethanreihe durch eine komplizierte zeit- und temperaturgestufte Formaldehydzugabe auf der Polyaminstufe, die dann durch Phosgenierung in das gewünschte Isocyanat überführt wird.

Nachteilig an allen diesen Vorgehensweisen ist, dass sie technisch aufwendig und/oder wenig effizient sind.

Aufgabe der vorliegenden Erfindung ist es daher, ein technisch einfaches und sicheres Verfahren bereit zu stellen, mit dem Polyisocyanate der Diphenylmethanreihe mit niedrigen Farbwerten hergestellt werden können. Es ist ebenfalls Aufgabe der vorliegenden Erfindung, ein einfaches Verfahren zur Herstellung von Polyaminen der Diphenylmethanreihe bereit zu stellen, aus denen Polyisocyanate der Diphenylmethanreihe mit niedrigen Farbwerten durch Phosgenierung hergestellt werden können.

Die Aufgabe wird erfindungsgemäß gelöst durch ein Verfahren zur Herstellung von Polyaminen der Diphenylmethanreihe, bei dem man
a) Anilin und Formaldehyd in Gegenwart eines sauren Katalysators zu Polyaminen umsetzt und danach
b) das Reaktionsgemisch in Gegenwart eines Alkohols neutralisiert und /oder das Reaktionsgemisch nach der Neutralisation mit einem Alkohol versetzt und /oder nach der Neutralisation die Phasen trennt und die organische Phase mit einer Base und einem Alkohol versetzt,
wobei das molare Verhältnis des Alkohols zum eingesetzten Formaldehyd
mindestens 0,02 beträgt.

Die Aufgabe wird ebenfalls erfindungsgemäß gelöst durch ein Verfahren zur Herstellung von Polyisocyanaten der Diphenylmethanreihe, bei dem man
a) Anilin und Formaldehyd in Gegenwart eines sauren Katalysators zu Polyaminen umsetzt und danach
b) das Reaktionsgemisch in Gegenwart eines Alkohols neutralisiert und /oder das Reaktionsgemisch nach der Neutralisation mit einem Alkohol versetzt und /oder nach der Neutralisation die Phasen trennt und die organische Phase mit einer Base und einem Alkohol versetzt,
   wobei das molare Verhältnis des Alkohols zum eingesetzten Formaldehyd mindestens 0,02 beträgt und danach
c) die Polyamine durch Phosgenierung zu den entsprechenden Polyisocyanaten umsetzt.

Das erfindungsgemäße Verfahren kann sowohl kontinuierlich als auch diskontinuierlich durchgeführt werden.

Mit dem erfindungsgemäßen Verfahren können Polyisocyanate mit niedrigen Farbwerten hergestellt werden. Als Farbwert wird hierbei die gemessene Extinktion einer Lösung von Polyisocyanat in Monochlorbenzol, enthaltend 2 Gew.-% Polyisocyanat, bei einer Schichtdicke von 10 mm und Raumtemperatur gegen Monochlorbenzol bei definierten Wellenlängen verstanden.

Das im erfindungsgemäßen Verfahren hergestellte Polyamin bzw. Polyamingemisch der Diphenylmethanreihe wird durch Kondensation von Anilin und Formaldehyd in Gegenwart eines sauren Katalysators erhalten (H.J. Twitchett, Chem. Soc. Rev. 3(2), 209 (1974), M.V. Moore in: Kirk-Othmer Encycl. Chem. Technol., 3^{rd}. Ed., New York, 2, 338-348 (1978)). Für das erfindungsgemäße Verfahren ist es dabei unerheblich, ob Anilin und Formaldehyd zunächst in Abwesenheit des sauren Katalysators vermischt werden und der saure Katalysator anschließend zugesetzt wird oder ob ein Gemisch aus Anilin und saurem Katalysator mit Formaldehyd zu Reaktion gebracht wird.

Geeignete Polyamingemische der Diphenylmethanreihe werden üblicherweise erhalten durch Kondensation von Anilin und Formaldehyd im Stoffmengenverhältnis 20 bis 1,6, bevorzugt 10 bis 1,8 sowie einem Stoffmengenverhältnis von Anilin und saurem Katalysator von 20 bis 1, bevorzugt 10 bis 2.

Formaldehyd wird technisch üblicherweise als wässrige Lösung eingesetzt. Es können jedoch auch andere methylengruppenliefernde Verbindungen wie z.B. Polyoxymethylenglykol, para-Formaldehyd oder Trioxan eingesetzt werden.

Als saure Katalysatoren haben sich starke organische und vorzugsweise anorganische Säuren bewährt. Geeignete Säuren sind beispielsweise Salzsäure, Schwefelsäure, Phosphorsäure, und Methansulfonsäure. Bevorzugt wird Salzsäure eingesetzt.

In einer bevorzugten Ausführungsform des Verfahrens wird Anilin und saurer Katalysator zunächst vereinigt. Dieses Gemisch wird, ggf. nach Abfuhr von Wärme, in einem weiteren Schritt mit Formaldehyd bei Temperaturen zwischen 20°C und 100°C, bevorzugt bei 30°C bis 70°C in geeigneter Weise vermischt und anschließend in einem geeigneten Verweilzeitapparat einer Vorreaktion zu unterzogen. Die Vorreaktion erfolgt bei Temperaturen zwischen 20°C bis 100°C, vorzugsweise im Temperaturbereich 30°C bis 80°C. Im Anschluss an Vermischung und Vorreaktion wird die Temperatur des Reaktionsgemisches in Stufen oder kontinuierlich und ggf. unter Überdruck auf eine Temperatur von 100°C bis 250°C, bevorzugt auf 100C bis 180°C, besonders bevorzugt auf eine Temperatur von 100°C bis 160 °C gebracht.

Es ist jedoch ebenfalls möglich in einer anderen Ausführungsform des Verfahrens Anilin und Formaldehyd zunächst in Abwesenheit des sauren Katalysators im Temperaturbereich von 5°C bis 130°C, bevorzugt von 40°C bis 100°C, besonders bevorzugt von 60°C bis 85°C, zu vermischen und so zur Reaktion zu bringen. Hierbei bilden sich Kondensationsprodukte aus Anilin und Formaldehyd (sog. Aminal). Im Anschluss an die Aminalbildung kann im Reaktionsgemisch vorhandenes Wasser durch Phasentrennung oder andere geeignete Verfahrensschritte, beispielsweise durch Destillation, entfernt werden. Das Kondensationsprodukt wird dann in einem weiteren Verfahrensschritt mit dem sauren Katalysator in geeigneter Weise vermischt und in einem Verweilzeitapparat bei 20°C bis 100°C, bevorzugt 30°C bis 80°C einer Vorreaktion unterzogen. Anschließend wird die Temperatur des Reaktionsgemisches in Stufen oder kontinuierlich und ggf. unter Überdruck auf eine Temperatur von 100°C bis 250°C, bevorzugt auf 100°C bis 180°C, besonders bevorzugt auf eine Temperatur von 100°C bis 160°C gebracht.

Die Umsetzung von Anilin und Formaldehyd in Gegenwart eines sauren Katalysators zu Polyaminen der Diphenylmethanreihe kann in Anwesenheit weiterer Stoffe (z.B. Lösungsmittel, Salze, organische und anorganische Säuren) geschehen.

Zur Aufarbeitung des sauren Reaktionsgemisches wird das Reaktionsgemisch mit einer Base neutralisiert. Nach dem Stand der Technik erfolgt die Neutralisation üblicherweise bei Temperaturen von beispielsweise 90 bis 100°C ohne Zusatz weiterer Substanzen. (H.J. Twitchett, Chem. Soc. Rev. 3(2), 223 (1974)). Als Basen sind beispielsweise geeignet die Hydroxide der Alkali- und Erdalkalielemente. Vorzugsweise kommt wässrige NaOH zur Anwendung.

Im erfindungsgemäßen Verfahren wird das saure Reaktionsgemisch in Gegenwart eines Alkohols neutralisiert und /oder das Reaktionsgemisch nach der Neutralisation mit einem Alkohol versetzt und /oder aus dem neutralisierten Reaktionsgemisch die wässrige Phase entfernt und die verbleibende organische Phase mit einer Base und mit einem Alkohol versetzt. Die Aufgabe des Alkohols besteht dabei darin, die Löslichkeit für Hydroxyl-Ionen in der organischen Phase zu erhöhen. Daher sind grundsätzlich alle Verbindungen, welche die Löslichkeit der Hydroxyl-Ionen und damit ihre Konzentration in der organischen Phase erhöhen, für das erfindungsgemäße Verfahren geeignet.

Geeignet sind insbesondere die Alkohole Methanol, Ethanol, n-Propanol, i-Propanol, Mono- und Diethanolamin und deren N-substituierte Derivate und Triethanolamin. Bevorzugt wird Methanol verwendet. Die positive Wirkung der lösungsvermittelnden Alkohole ist nicht auf den Einsatz der Reinstoffe beschränkt. Im erfindungsgemäßen Verfahren können auch Gemische von Alkoholen verwendet werden.

In einer Ausführungsform des erfindungsgemäßen Verfahrens wird das saure Reaktionsgemisch aus der Umsetzung von Anilin und Formaldehyd mit einer Base in Gegenwart eines Alkohols neutralisiert.

Die Neutralisation erfolgt vorteilhaft in der Weise, dass das saure Reaktionsgemisch der Anilin/Formaldehydkondensation mit der Base und dem entsprechenden Alkohol vermischt und einem Verweilzeitapparat zugeführt wird. Bei geeigneten Verweilzeitapparaten (z.B. Rührkessel) kann die Vermischung von saurem Kondensationsgemisch, Base und Alkohol auch direkt im Verweilzeitapparat erfolgen.

Die Einspeisung des löslichkeitsvermittelnden Alkohols muss nicht notwendigerweise erst auf der Stufe der Neutralisation erfolgen. Vielmehr ist es auch möglich, den Alkohol am Prozessanfang mit einem der Edukte (Anilin, Formalin, Salzsäure) in den Prozess einzutragen. Auch die direkte Einspeisung des Alkohols in eine beliebige Stelle der säurekatalysierten Reaktion von Anilin und Formalin ist möglich. Es ist auch möglich, dass der Alkohol erst im Anschluss an die Neutralisation zugegeben wird (im kontinuierlichen Verfahren beispielsweise in einem nachgeschalteten Verweilzeitapparat) und für eine ausreichende Verweilzeit mit dem neutralisierten Reaktionsgemisch in Kontakt kommt. Es ist auch möglich, den Alkohol in mehreren Portionen an verschiedenen Orten oder zu verschiedenen Zeiten im Verfahren jeweils anteilig zuzugeben.

Die Verweilzeit des Reaktionsgemisches in Gegenwart des Alkohols in dem Neutralisationsapparat bzw. im nachgeschalteten Verweilzeitapparat liegt vorzugsweise bei ≥ 0,1 Minute, besonders bevorzugt 0,1 bis 180 Minuten, ganz besonders bevorzugt 2 bis 120 Minuten, insbesondere ganz besonders bevorzugt 5 bis 60 Minuten. Um das Sieden unterhalb bzw. bei einer gewünschten Temperatur zu verhindern, muss der Verfahrensschritt gegebenenfalls unter erhöhtem Druck durchgeführt werden.

Die zur Neutralisation eingesetzte Base wird vorzugsweise in Mengen von größer 100%, bevorzugt 101 bis 140 %, vorzugsweise 105 bis 120 % der für die Neutralisation des eingesetzten sauren Katalysators stöchiometrisch benötigten Menge eingesetzt. Der Alkohol bzw. das Alkoholgemisch wird bezogen auf den für die Kondensation verwendeten Formaldehyd in einem molaren Verhältnis von mindestens 0,02, bevorzugt 0,025 bis 100, besonders bevorzugt 0,03 bis 50, ganz besonders bevorzugt 0,04 bis 10, insbesondere ganz besonders bevorzugt 0,05 bis 5 eingesetzt. Der Effekt der Neutralisation unter Zusatz eines Alkohols auf die MDI-Farbe wird verstärkt, wenn in dem Neutralisations- bzw. dem nachgeschaltetem Verweilzeitapparat für eine ausreichende Durchmischung der organischen und wässrigen Phase gesorgt wird. Dies kann durch Anwendung der in der Technik bekannten Methoden beispielsweise durch statische oder dynamische Mischer oder Erzeugung von Turbulenz erfolgen.

Im Anschluss an die Neutralisation wird in einem Scheidebehälter die organische von der wässrigen Phase getrennt. Sollte eine Phasentrennung durch den Einsatz hoher Mengen an lösungsvermittelndem Alkohol nicht möglich sein, kann die Phasentrennung durch gezielten Zusatz von Wasser ausgelöst werden. Alternativ ist es ebenfalls möglich, den Alkohol zunächst durch geeignete Methoden aus dem neutralisierten Gemisch zu entfernen, beispielsweise durch Destillation, und dann die Phasentrennung vorzunehmen. Die nach Abtrennung der wässrigen Phase verbleibende produktenthaltende organische Phase wird weiteren Aufarbeitungsschritten unterzogen (z.B. Wäsche) und anschließend von überschüssigem Anilin und anderen im Gemisch vorhandenen Stoffen (z.B. weiteren Lösungsmitteln) durch geeignete Verfahren wie z.B. Destillation, Extraktion oder Kristallisation befreit.

In einer alternativen Ausführungsform des erfindungsgemäßen Verfahrens wird die Neutralisation nach dem Stand der Technik, beispielsweise bei einer Temperatur von 90 bis 100°C durchgeführt. Anschließend wird die Trennung der wässrigen und der organischen Phase nach einem der üblichen Verfahren, beispielsweise in einer Scheideflasche, vorgenommen. Nach der Phasentrennung wird die organische Phase der neutralisierten Reaktionsmischung in einem Verweilzeitbehälter mit einer Base, bevorzugt wässriger Natronlauge, vermischt und mit einem Alkohol versetzt.

Die Base wird in Mengen von größer 1%, bevorzugt 2 bis 140%, vorzugsweise 5 bis 120% der für die Neutralisation des für die Kondensationsreaktion verwendeten sauren Katalysators stöchiometrisch benötigten Menge eingesetzt. Der Alkohol oder das Alkoholgemisch wird bezogen auf den für die Kondensation eingesetzten Formaldehyd in einem molaren Verhältnis von mindestens 0,02, bevorzugt 0,025 bis 100, besonders bevorzugt 0,03 bis 50, ganz besonders bevorzugt 0,04 bis 10, insbesondere ganz besonderes bevorzugt 0,05 bis 5 eingesetzt. Die Behandlung der organischen Phase der neutralisierten Reaktionsmischung erfolgt dabei beispielsweise in der Weise, dass die organische Phase wahlweise mit der Base, dem Alkohol bzw. Alkoholgemisch oder beiden in einem Mischaggregat vermischt und anschließend dem Verweilzeitapparat zugeführt wird (beispielsweise Rührkessel, Rührkesselkaskade, Strömungsrohr oder Umpumpreaktor). Bei geeigneten Verweilzeitapparaten kann die Vermischung von organischer Phase mit der Base und dem Alkohol bzw. Alkoholgemisch auch direkt im Verweilzeitapparat erfolgen.

Die Behandlung der organischen Phase der neutralisierten Reaktionsmischung mit der Base und dem Alkohol oder Alkoholgemisch wird vorzugsweise für eine Verweilzeit von ≥ 0,1 Minute, besonders bevorzugt 0,1 bis 180 Minuten, ganz besonders bevorzugt 2 bis 120 Minuten, insbesondere ganz besonders bevorzugt 5 bis 60 Minuten vorgenommen. Um das Sieden unterhalb bzw. bei einer gewünschten Temperatur zu verhindern, muss der Verfahrensschritt gegebenenfalls unter erhöhtem Druck durchgeführt werden.

Der Effekt auf die Farbe der Polyisocyanate der Diphenylmethanreihe wird verstärkt, wenn in dem Verweilzeitbehälter für eine ausreichende Durchmischung der organischen und wässrigen Phase gesorgt wird. Dies kann durch Anwendung der in der Technik bekannten Methoden, beispielsweise durch statische oder dynamische Mischer oder Erzeugung von Turbulenz erfolgen. Nach der Behandlung der organischen Phase mit dem Alkohol, die bevorzugt in Gegenwart der Base durchgeführt wird, wird eine weitere Phasentrennung vorgenommen und die organische Phase den weiteren Aufarbeitungsschritten zugeführt. Sollte eine Phasentrennung durch den Einsatz hoher Mengen an lösungsvermittelndem Alkohol nicht möglich sein, kann die Phasentrennung durch gezielten Zusatz von Wasser ausgelöst werden. Es ist aber ebenso möglich, den Alkohol zunächst durch geeignete Methoden aus dem neutralisierten Gemisch zu entfernen, beispielsweise durch Destillation, und dann die Phasentrennung vorzunehmen. Weiterhin ist es möglich, die die Base enthaltende wässrige Phase in die Neutralisation des sauren Reaktionsgemisches aus der Kondensation von Anilin und Formaldehyd einzutragen, gegebenenfalls nach Zugabe von Wasser, um die gewünschte Basenkonzentration einzustellen.

Das so erhaltene Polyamin oder Polyamingemisch der Diphenylmethanreihe wird nach den bekannten Methoden mit Phosgen in einem inerten organischen Lösungsmittel zu den entsprechenden Isocyanaten umgesetzt. Das Molverhältnis von Roh-MDA zu Phosgen wird zweckmäßigerweise so bemessen, dass pro Mol NH₂-Gruppe 1 bis 10 Mol, vorzugsweise 1,3 bis 4 Mol Phosgen in der Reaktionsmischung vorliegen. Als inerte Lösungsmittel haben sich chlorierte, aromatische Kohlenwasserstoffe wie z.B. Monochlorbenzol, Dichlorbenzole, Trichlorbenzole, die entsprechenden Toluole und Xylole sowie Chlorethylbenzol bewährt. Insbesondere Anwendung finden als inerte organische Lösungsmittel Monochlorbenzol, Dichlorbenzol oder Mischungen dieser Chlorbenzole. Die Menge an Lösungsmittel wird zweckmäßigerweise so bemessen, dass die Reaktionsmischung einen Isocyanatgehalt von 2 bis 40 Gew.-%, vorzugsweise zwischen 5 uns 20 Gew.-%, bezogen auf das Gesamtgewicht der Reaktionsmischung, aufweist. Nach beendeter Phosgenierung wird dem Reaktionsmischung das überschüssige Phosgen, das inerte organische Lösungsmittel oder Mischungen davon durch Destillation abgetrennt.

Die nach dem erfindungsgemäßen Verfahren hergestellten Roh-MDI besitzen eine deutlich reduzierte Färbung.

Die Erfindung soll anhand der nachfolgenden Beispiele näher erläutert werden.

### Beispiel 1 (Vergleichsbeispiel)

Zu 200 g Anilin wurden unter Stickstoffbeschleierung bei 80°C innerhalb von 20min zugleich 1011 g Anilin und 611g einer 32 %igen wässrigen Formaldehydlösung getropft. Nach der Zugabe wurde noch 10 min gerührt und anschließend eine Phasentrennung bei 70 bis 80°C vorgenommen. Von der organischen Phase wurde eine Menge von 300 g in einer Stickstoffatmosphäre auf 35°C temperiert und anschließend bei dieser Temperatur innerhalb von 30 min mit der restlichen organischen Phase und 373 g einer 32 %igen wässrigen Salzsäure versetzt. Nach beendeter Zugabe und einer 30 minütigen Nachrührzeit bei dieser Temperatur wurde während 10min auf 60°C aufgeheizt und 30min bei dieser Temperatur gehalten. Anschließend wurde innerhalb von 30 min auf Rückflusstemperatur aufgeheizt (ca. 105°C) und 10 h unter Rückfluss gerührt. Zu 684 g des so erhaltenen sauren Kondensationsgemisches wurden 123 g einer 50 %igen wässrigen Natronlauge und 265 ml siedendes Wasser gegeben. Nach 30 min Rühren unter Rückfluss (ca. 105°C) wurde bei 80-90°C eine Phasentrennung vorgenommen und die organische Phase weitere 2× mit je 800 ml siedendem Wasser gewaschen. Die organische Phase wurde anschließend unter vermindertem Druck vom überschüssigen Anilin befreit. Von dem so erhaltenen Polyamin wurden 50 g in 255 ml Chlorbenzol gelöst, auf 55°C erwärmt und innerhalb von 10 s unter intensivem Rühren zu einer auf 0°C temperierten Lösung von 105 g Phosgen in 310 ml Chlorbenzol gegeben. Die Suspension wurde unter Durchleiten von Phosgen innerhalb von 45 min auf 100°C und anschließend während 10 min auf Rückflusstemperatur aufgeheizt. Nach weiteren 10 min bei dieser Temperatur wurde das Lösungsmittel unter vermindertem Druck bis zu einer Sumpftempratur von 100°C abdestilliert. Das rohe Isocyanat wurde anschließend in einer Destillationsappartur bei einem Druck von 4 bis 6 mbar durch ein auf 260°C erwärmtes Heizbad bis zum ersten Produktübergang erhitzt und daraufhin innerhalb von 5 min auf Raumtemperatur abgekühlt. Von dem so erhaltenen Isocyanat wurde 1,0 g in Chlorbenzol gelöst und mit Chlorbenzol zu 50 ml verdünnt. Die so erhaltene Lösung besaß bei 430 nm einen Extinktionswert von 0,198 gegen Chlorbenzol.

### Beispiel 2 (erfindungsgemäß)

Aus 838 g Anilin, 420 g einer 32 %igen wässrigen Formaldehydlösung und 256 g 32 %igen Salzsäure wurde gemäß Beispiel 1 ein saures Kondensationsgemisch hergestellt. 247 g dieses sauren Kondensationsgemisches wurden mit 74 g 32 %iger wässriger Natronlauge und 74 g Methanol versetzt und 30 min bei 105°C gerührt. Nach Abkühlen auf 60°C wurde die Reaktionsmischung mit 700 ml Wasser versetzt und nach der Phasentrennung, wie in Beispiel 1 beschrieben, die organische Phase mit Wasser gewaschen und durch Destillieren unter vermindertem Druck vom Anilin befreit. Das resultierende Polyamin wurde analog Beispiel 1 mit Phosgen in das entsprechende Isocyanat überführt. Die Extinktion bei 430 nm gemäß der in Beispiel 1 beschriebenen Methode betrug 0,172 gegen Chlorbenzol.

### Beispiel 3 (erfindungsgemäß)

Aus 838 g Anilin, 420 g einer 32 %igen wässrigen Formaldehydlösung und 256 g 32 %igen Salzsäure wurde gemäß Beispiel 1 ein saures Kondensationsgemisch hergestellt. 222 g dieses sauren Kondensationsgemisches wurden mit 66 g 32 %iger wässriger Natronlauge und 95 g Ethanol versetzt und 30 min bei 105°C gerührt. Nach Abkühlen auf 60°C wurde die Reaktionsmischung mit 700 ml Wasser versetzt und nach der Phasentrennung, wie in Beispiel 1 beschrieben, die organische Phase mit Wasser gewaschen und durch Destillieren unter vermindertem Druck vom Anilin befreit. Das resultierende Polyamin wurde analog Beispiel 1 mit Phosgen in das entsprechende Isocyanat überführt. Die Extinktion bei 430 nm gemäß der in Beispiel 1 beschriebenen Methode betrug 0,164 gegen Chlorbenzol.

## Patentansprüche

1. Verfahren zur Herstellung von Polyaminen der Diphenylmethanreihe, bei dem man
a) Anilin und Formaldehyd in Gegenwart eines sauren Katalysators zu Polyaminen umsetzt und danach
b) das Reaktionsgemisch in Gegenwart eines Alkohols neutralisiert und/oder das Reaktionsgemisch nach der Neutralisation mit einem Alkohol versetzt und/oder nach der Neutralisation die Phasen trennt und die organische Phase mit einer Base und einem Alkohol versetzt,
wobei das molare Verhältnis des Alkohols zum eingesetzten Formaldehyd mindestens 0,02 beträgt.

2. Verfahren nach Anspruch 1, bei dem man das Reaktionsgemisch mit wässriger Natronlauge neutralisiert.

3. Verfahren nach einem der Ansprüche 1 bis 2, bei dem man Methanol, Ethanol, n-Propanol, iso-Propanol, Monoethanolamin oder Diethanolamin oder deren N-substituierte Derivate oder Triethanolamin oder Gemische daraus als Alkohol einsetzt.

4. Verfahren zur Herstellung von Polyisocyanaten der Diphenylmethanreihe, bei dem man
a) Anilin und Formaldehyd in Gegenwart eines sauren Katalysators zu Polyaminen umsetzt und danach
b) das Reaktionsgemisch in Gegenwart eines Alkohols neutralisiert und/oder das Reaktionsgemisch nach der Neutralisation mit einem Alkohol versetzt und/oder nach der Neutralisation die Phasen trennt und die organische Phase mit einer Base und einem Alkohol versetzt,
wobei das molare Verhältnis des Alkohols zum eingesetzten Formaldehyd mindestens 0,02 beträgt und danach
c) die Polyamine durch Phosgenierung zu den entsprechenden Polyisocyanaten umsetzt.

5. Verfahren nach Anspruch 4, bei dem man das Reaktionsgemisch mit wässriger Natronlauge neutralisiert.

6. Verfahren nach einem der Ansprüche 4 bis 5, bei dem man Methanol, Ethanol, n-Propanol, iso-Propanol, Monoethanolamin oder Diethanolamin oder deren N-substituierte Derivate oder Triethanolamin oder Gemische daraus als Alkohol einsetzt.
